Europäisches Patentamt

European Patent Office

Office européen des brevets

(11) Numéro de publication : **0 077 264 B1**

# (12) FASCICULE DE BREVET EUROPÉEN

(45) Date de publication du fascicule du brevet :
23.03.88

(51) Int. Cl.⁴ : **A 61 K 9/32**, A 23 K 1/00, A 61 K 47/00

(21) Numéro de dépôt : **82401838.6**

(22) Date de dépôt : **06.10.82**

(54) Nouvelle composition pour l'enrobage des aliments et des médicaments et granulés ainsi enrobés.

(30) Priorité : 08.10.81 FR 8118954

(43) Date de publication de la demande :
20.04.83 Bulletin 83/16

(45) Mention de la délivrance du brevet :
23.03.88 Bulletin 88/12

(84) Etats contractants désignés :
AT BE CH DE FR GB IT LI LU NL SE

(56) Documents cités :
FR-A- 2 401 621
Le dossier contient des informations techniques
présentées postérieurement au dépôt de la demande
et ne figurant pas dans le présent fascicule.

(73) Titulaire : **A.E.C. - Société de Chimie Organique et Biologique**
**Avenue Edouard Vaillant**
**F-03600 Commentry (FR)**

(72) Inventeur : **Autant, Pierre**
**14 rue de Pourcheroux**
**F-03600 Commentry (FR)**
Inventeur : **Cartillier André**
**Champfromenteau**
**F-03600 Commentry (FR)**
Inventeur : **Quentin, Jean-Pierre**
**35 rue Joliot Curie**
**F-69005 Lyon (FR)**

(74) Mandataire : **Pilard, Jacques et al**
**RHONE-POULENC INTERSERVICES Service Brevets**
**Pharma 25, Quai Paul Doumer**
**F-92408 Courbevoie Cedex (FR)**

EP 0 077 264 B1

Jouve, 18, rue St-Denis, 75001 Paris, France

**0 077 264**

## Description

La présente invention concerne une composition pour l'enrobage des aliments et des médicaments qui est stable dans un milieu dont le pH est supérieur ou égal à 5,5 et qui permet la libération de la substance biologiquement active dans un milieu dont le pH est inférieur ou égal à 3,5.

En particulier, lorsqu'on administre à des ruminants certaines substances biologiquement actives (médicaments, aliments enrichis), il se produit lors du passage dans le rumen une destruction enzymatique de ces substances favorisée par le temps de séjour (quelques heures à plusieurs jours) et par le pH (compris entre 5 et 6).

Il importe donc de pouvoir protéger ces substances biologiquement actives par des enrobages qui soient stables à un pH supérieur ou égal à 5, c'est-à-dire qui soient stables dans la panse des ruminants, qui résistent à la dégradation par les microorganismes et qui permettent la libération des substances biologiquement actives dans une partie de l'appareil digestif plus particulièrement dans la caillette dont le pH est inférieur ou égal à 3,5. Alors que la durée de la protection dans la panse doit être relativement longue (plusieurs heures à quelques jours), la libération de la substance active doit s'effectuer en un temps relativement court (quelques minutes à 1 ou 2 heures).

Pour obtenir un tel résultat, il est avantageux de disposer de polymères, utilisables pour l'enrobage des substances actives, dont la structure est telle qu'ils sont insolubles dans le rumen à un pH compris entre 5 et 6, mais solubles, dispersés ou fortement gonflés ensuite dans la caillette à un pH inférieur à 3,5, pour libérer le produit actif.

Dans ce domaine, il a été proposé d'utiliser, entre autres, des copolymères de l'anhydride maléique avec un autre monomère, modifiés par action d'une diamine primaire-tertiaire sur les groupements anhydrides, formant ainsi des groupes imides aminés qui apportent la solubilité souhaitée (brevet français 1 536 774). Des dérivés cellulosiques aminés sont également connus ; ils sont obtenus à partir d'un dérivé non saturé de cellulose (éther, ester) sur lequel on fait réagir un composé azoté contenant un atome d'hydrogène mobile, tel que pipéridine, morpholine, amine secondaire (brevet français 69 30562/2081320).

Par ailleurs, dans les brevets britanniques 1 137 214, australien 454 117 et belge 865 654 et la demande sud-africaine 70 04813 ainsi que dans les brevets français 74 33108/2246572 et américain 3 341 505 sont décrits des copolymères de :

a) un monomère éthylénique neutre comme l'acrylate ou le méthacrylate de méthyle, le styrène, l'acrylonitrile, l'acétate de vinyle, et

b) un monomère éthylénique portant un groupement azoté basique comme l'acrylate ou le méthacrylate de diéthylaminoéthyle, l'acrylate ou le méthacrylate de t.butylaminoéthyle, le méthacrylate de morpholinoéthyle ou les vinylpyridines.

Pour enrober des aliments destinés à la nourriture des ruminants, il a été proposé d'utiliser des copolymères styrène-vinyl-pyridine contenant des substances hydrophobes choisies parmi les acides gras contenant 12 à 32 atomes de carbone ou des acides polycarboxyliques comprenant 10 à 22 atomes par groupe carboxyle qui améliorent la protection en diminuant la susceptibilité globale de la pellicule d'enrobage aux milieux aqueux de caractère faiblement acide (brevet français 78 23966/2401620). Dans de telles compositions d'enrobage, la substance hydrophobe permet de diminuer la mouillabilité du polymère mais reste sans influence sur la libération du principe actif au milieu acide.

Dans le brevet français FR 78 23698 (2 401 621), il a été proposé des compositions d'enrobage constituées par une enveloppe continue d'un polymère hydrophobe insoluble en milieu aqueux contenant en dispersion une substance minérale ou une substance organique choisie parmi les particules de polymères réticulés contenant 3 à 14 % d'azote. Cependant de telles compositions ne permettent pas d'obtenir des produits enrobés dont la pH sensibilité est satisfaisante.

Il a maintenant été trouvé, et c'est ce qui fait l'objet de la présente invention, que l'on obtient une composition homogène permettant l'enrobage d'une substance biologiquement active en associant un copolymère non réticulé sensible aux variations du pH à un polymère hydrophobe non hydrosoluble insensible aux variations du pH dans lequel le copolymère non réticulé est soluble et éventuellement à un acide organique, le second polymère améliorant la libération de la substance active à un pH compris entre 1 et 2,5 et diminuant son extractibilité en milieu aqueux.

Le copolymère non réticulé sensible aux variations du pH est choisi parmi les copolymères du styrène avec les vinylpyridines telles que la vinyl-2 ou la vinyl-4 pyridine ou les alcoylvinylpyridines comme la méthyl-2 vinyl-5 pyridine. Dans ces copolymères, la teneur en styrène est comprise entre 5 et 70 % et de préférence entre 5 et 50 %, les meilleurs résultats étant obtenus avec des copolymères dont la teneur en styrène est comprise entre 10 et 40 %.

Le copolymère non réticulé utilisable dans l'enrobage selon la présente invention est caractérisé par un poids moléculaire compris entre 100 000 et 700 000 et de préférence entre 150 000 et 500 000

Selon la présente invention, le polymère hydrophobe insensible aux variations du pH, entrant dans l'enrobage, doit présenter comme caractère essentiel celui d'être insoluble dans l'eau.

Par ailleurs, il est sans importance qu'il y ait ou non compatibilité du polymère hydrophobe non hydrosoluble avec le copolymère styrène-vinylpyridine. La compatibilité peut être définie par l'aspect

2

d'un film obtenu à partir d'une solution homogène d'un mélange en quantités égales du copolymère et du polymère : il y a incompatibilité lorsque l'observation du film à l'œil nu montre la présence de zones correspondant à chacun des constituants. Parmi les polymères hydrophobes non hydrosolubles, qui peuvent être utilisés pour la préparation de la composition d'enrobage selon l'invention, peuvent être cités : l'acétobutyrate de cellulose, l'éthylcellulose, le propionate de cellulose, le caoutchouc chloré, la polycaprolactóne ou le polystyrène. Les meilleurs résultats sont obtenus avec l'acétobutyrate de cellulose.

L'adjonction du polymère hydrophobe non hydrosoluble dans la composition permet de réaliser l'enrobage de substances actives en utilisant des quantités moins importantes d'enrobant tout en ayant un enrobage de qualité correcte en particulier lorsque l'encapsulation s'effectue selon la technique de lit fluidisé. La quantité d'enrobant peut être jusqu'à 50 % inférieure à celle qui serait nécessaire sans adjonction de polymère.

La quantité de polymère ajouté est fonction de la sensibilité en milieu acide du copolymère et de sa composition. Le polymère hydrophobe non hydrosoluble peut représenter de 10 à 75 % en poids du mélange des polymères.

En utilisant comme polymère hydrophobe non hydrosoluble, l'acétobutyrate de cellulose, les meilleurs résultats sont obtenus lorsque la quantité de polymère ajouté représente 20 à 40 % en poids du mélange des polymères.

Par ailleurs, l'adjonction du polymère hydrophobe non hydrosoluble conduit généralement à une libération plus importante de la substance active dans un milieu dont le pH est voisin de 1,5 et permet d'obtenir une teneur plus élevée de la substance active dans le sang des animaux traités.

Il a également été trouvé que la composition d'enrobage pouvait être améliorée en incorporant un diacide organique (tel que les acides phtalique, oxalique, malonique ou succinique) ou l'acide benzoïque. Cette addition facultative permet une amélioration de la libération de la substance active jusqu'à pH 3. Cependant l'addition d'un acide tel que défini précédemment favorise également l'extraction de la substance active en milieu aqueux. En conséquence la quantité d'acide ajouté conditionne la quantité de polymère non hydrosoluble qui doit être introduit dans la composition afin de conserver une faible extractibilité en milieu aqueux et une amélioration de la libération de la substance active en milieu acide. La quantité d'acide ajouté peut représenter jusqu'à 50 % en poids du copolymère mis en œuvre.

Selon la présente invention, d'autres adjuvants peuvent être introduits dans la composition d'enrobage afin d'en faciliter sa mise en œuvre selon les techniques utilisées. Parmi ces adjuvants peuvent être cités des plastifiants, tels que le phtalate de butyle ou d'octyle l'adipate de dibutyle ou de di(éthyl-2 hexyle) ou des agents antistatiques, tels que des sels d'ammonium quaternaires ou la N-acétyléthanolamine, ou des colorants.

La composition d'enrobage est généralement obtenue en dissolvant le copolymère et le polymère non hydrosoluble dans un solvant organique tel que le tétrahydrofuranne ou dans un mélange de solvants organiques tel que le mélange acétone-dichloréthane. A la solution obtenue, peuvent être ajoutés d'autres adjuvants tels que des plastifiants ou des colorants, ou en particulier lorsque l'encapsulation est effectuée en lit d'air fluidisé, des agents antistatiques.

Les compositions selon la présente invention sont particulièrement utiles pour l'enrobage de substances thérapeutiques et nutritives diverses, telles que des médicaments, des vitamines ou des aminoacides, destinées à être administrées par voie orale à des ruminants. Ces substances enrobées sont généralement mélangées à la nourriture des animaux.

Ces substances enrobées se présentent de préférence sous forme de granulés constitués d'un noyau central entouré d'une pellicule continue de la composition d'enrobage. Cependant, les substances actives peuvent aussi être dispersées dans la composition d'enrobage. En général, la substance active représente 60 à 95 % en poids du granulé.

La présente invention concerne également les granulés constitués de substances biologiquement active enrobée dans la composition d'enrobage.

Les granulés peuvent être obtenus par application des techniques habituelles d'enrobage, telles que l'encapsulation en lit fluidisé ou la coacervation, qui permettent de déposer une pellicule continue de l'enrobant autour d'un noyau qui est constitué essentiellement par la substance active et qui peut être sous forme de granulés.

Les granulés obtenus doivent présenter des propriétés mécaniques telles qu'ils soient stables au stockage et lors des manipulations, qu'ils ne se détériorent pas lors de la confection des aliments pour les animaux et qu'ils ne soient pas détruits lors de leur absorption par les animaux et en particulier lors de la mastication par écrasement ou broyage.

La taille des granulés sera fonction de l'utilisation qui doit en être faite et plus particulièrement sera déterminée en fonction de l'animal auquel ils sont destinés. Il est possible d'enrober des granulés dont la taille est comprise entre 0,5 et 5 mm.

D'un intérêt tout particulier sont les granulés qui contiennent comme principe actif la méthionine dont le rôle est très important dans l'alimentation des animaux et plus précisément des ruminants.

Les exemples suivants, donnés à titre non limitatif, illustrent les compositions d'enrobage selon l'invention et leur utilisation pour la préparation de substances actives enrobées.

Dans les exemples, les propriétés des produits sont déterminées à partir des essais suivants :

1) extraction en milieu acide in vitro

On fait incuber 0,5 g du produit à étudier pendant 15 minutes, 30 minutes ou 1 heure dans 50 cm³ de milieu KCl/HCl amené préalablement au pH désiré (1,5 ou 3) maintenu non agité dans un bain thermostaté à 40 °C.

Lorsque le temps d'incubation est écoulé, le liquide d'extraction est filtré. Le produit actif dissout est dosé selon une méthode appropriée. Cette valeur rapportée à la quantité de produit initialement introduit donne le taux de libération.

Dans le cas où la substance active est la méthionine, la méthionine dissoute est déterminée quantitativement par iodométrie selon la méthode dans « New and Non-Official Remedies », Lippincott (1952).

2) résistance au rumen

Des prises d'essai d'environ 0,5 g de granulés à étudier sont introduites dans des sachets de nylon dont le vide de maille est de 300 × 300 µm. Les sachets sont mis à incuber pendant 48 heures dans le rumen de brebis fistulées. Les sachets sont ensuite récupérés et lavés. La substance active restante est quantitativement déterminée selon une méthode appropriée.

3) détermination des taux sanguins

L'efficacité relative de différents produits est mesurée par leur faculté d'élever le taux de substance active dans le sang lors de tests de supplémentation standardisés.

Dans le cas de la méthionine, on administre directement dans le rumen de brebis fistulées, des doses bi-journalières de 6 g d'équivalent méthionine. Ce régime de supplémentation est maintenu pendant 7 jours et des prélèvements sanguins sont effectués les 6ème et 7ème jours. La méthionine est déterminée sur le sang total, l'analyse quantitative étant faite selon la méthode de STEIN et MOORE.

Afin de vérifier la différence d'efficacité de deux formules, des tests croisés sont effectués sur plusieurs animaux. Après chaque période de supplémentation, on laisse s'écouler une période témoin de 7 jours sans apport de substance active à la fin de laquelle on vérifie que son taux sanguin est revenu à son niveau initial.

## Exemple 1

On enrobe 400 g de méthionine granulée sous forme de particules sphériques titrant 98 % dont le diamètre est compris entre 0,5 et 0,63 mm, selon la technique du lit fluidisé, avec 72 g d'un enrobage constitué de 70 parties de copolymère vinyl-2 pyridine-styrène (70-30) et de 30 parties d'acétobutyrate de cellulose.

On obtient des granulés dont les caractéristiques sont les suivantes :

| | |
|---|---|
| — titre en méthionine | 75,9 % |
| — extractible dans l'eau | 15,4 % après 16 heures |
| | 23,7 % après 48 heures |
| — résistance au rumen | 78,8 % après 48 heures |
| — extractible en milieu acide | à pH 1,5 |

71 % après 15 minutes ; 98 % après 30 minutes ; 100 % après 1 heure

A titre de comparaison des granulés préparés à partir de 400 g de méthionine et de 72 g de copolymère vinyl-2 pyridine-styrène présentent les caractéristiques suivantes :

| | |
|---|---|
| — titre en méthionine | 73 % |
| — extractible dans l'eau | 20,3 % après 16 heures |
| | 31,3 % après 48 heures |
| — résistance au rumen | 70 % après 48 heures |
| — extractible en milieu acide | à pH 1,5 |

42 % après 15 minutes ; 71 % après 30 minutes ; 85 % après 1 heure

Les granulés obtenus selon la présente invention ont été testés sur brebis fistulées. La méthionine enrobée est introduite dans le rumen par la fistule. Les animaux reçoivent une dose de 6 g/jour ou de 12 g/jour pendant 5 jours, puis la semaine suivante les animaux ne reçoivent rien (semaine témoin). L'estimation de la méthionine absorbée se fait par mesure de l'accumulation au niveau sanguin en fin de semaine. Le taux de méthionine sanguin est exprimé en mg de méthionine pour 100 g de sang. A la dose de 6 g/jour, le taux sanguin est de 6,9 mg/100 g de sang et à la dose de 12 g/jour, il est de 13,4 mg/100 g de sang, alors que, lorsque la méthionine est donnée libre, les taux sanguins sont respectivement de 1,32 et 1,61 mg pour 100 g de sang.

Il en résulte que le taux de méthionine sanguin est multiplié par 8,3 à la dose de 12 g/jour et par 5,3 à la dose de 6 g/jour.

La composition d'enrobage est préparée de la manière suivante :

On disperse 50,4 g de copolymère vinyl-2 pyridine-styrène (70-30) et 21,6 g d'acétobutyrate de cellulose dans 1 200 cm³ de tétrahydrofuranne, puis on ajoute 1,5 g de phtalate de butyle et 0,07 g de colorant.

Lorsque la dissolution est totale on ajoute de la N-acétyléthanolamine en quantité suffisante pour que la résistivité de la solution soit inférieure ou égale à $10^6\Omega$ cm.

Cette solution est alors pulvérisée sur la méthionine à enrober dans un appareil permettant l'enrobage en lit d'air fluidisé.

Le copolymère vinyl-2 pyridine-styrène, utilisé dans la préparation de l'enrobage, est obtenu de la manière suivante :

Dans un réacteur de 25 litres, on dissout 14 g de méthylcellulose dans 9 334 g d'eau distillée. La solution est chauffée à 60 °C puis on ajoute, en agitant à une vitesse de 150 tours/minute, un mélange de 1 200 g de styrène, de 2 800 g de vinyl-2 pyridine et de 40 g d'azobi-isobutyronitrile.

On maintient l'agitation et la température (60-63 °C) pendant 4 heures 30. La suspension est alors essorée. Le polymère recueilli est lavé à l'eau distillée puis est séché à 60 °C sous pression réduite (100 mm de mercure ; 13,3 kPa) jusqu'à poids constant.

On obtient ainsi 3,630 kg de copolymère styrène-vinyl-2 pyridine (28,6-71,4) qui a une viscosité spécifique de 0,520 (détermination en solution dans le diméthylformamide à 5 g/litre à 23 °C) et dont la masse moléculaire en poids est voisine de 410 000.

Le taux de polymérisation est de 91 %.

L'acétobutyrate de cellulose présente les caractéristiques suivantes :

Poids moléculaire moyen : 57 000

Taux de groupements

— acétate 1,2
— butyrate 1,8

par motif cellulosique

## Exemple 2

On opère comme dans l'exemple 1, mais en utilisant 1 200 g de méthionine granulée titrant environ 98 %, on obtient ainsi des granulés dont les caractéristiques sont les suivantes :

— titre en méthionine 77,5 %
— extractible dans l'eau à 20 °C 10,7 % après 16 heures
21,5 % après 48 heures
— résistance au rumen 84,1 % après 48 heures
— extractible en milieu acide à pH 1,5
94 % après 15 minutes ; 98 % après 30 minutes ; 100 % après 1 heure

Ces granulés sont soumis à un test de mastication chez la brebis fistulée. La méthionine protégée est administrée pendant 1 semaine dans la fistule et pendant 1 semaine par addition à l'aliment concentré. Entre ces deux semaines, se situe une semaine correspondant à une période témoin (absence de la supplémentation en méthionine).

La différence du taux de méthionine dans le sang est due à l'action de la mastication. Un abaissement de 13 à 15 % du taux de méthionine dans le sang est ainsi enregistré.

Les granulés ainsi obtenus sont essayés dans la préparation d'un aliment pour poussins à 0,2 % (préparation de 100 kg d'aliment dans un mélangeur « GONDARD »). Le pourcentage de méthionine extractible à l'eau dans l'aliment fabriqué correspond à celui obtenu pour la méthionine enrobée initiale ; en effet, en 2 heures, le pourcentage de méthionine extractible à l'eau (par rapport à la méthionine présente) représente 3,8 % pour l'aliment alors qu'il est de 4,4 % pour la méthionine enrobée initiale.

## Exemple 3

On enrobe 400 g de méthionine granulée sous forme de particules sphériques titrant 98 % dont le diamètre est compris entre 0,5 et 0,63 mm, selon la technique du lit fluidisé, avec 120 g d'un enrobage constitué de 70 parties de copolymère vinyl-2 pyridine-styrène (70-30) et de 30 parties d'acétobutyrate de cellulose.

Par ailleurs, on réalise, dans les mêmes conditions un produit témoin dont l'enrobage est constitué de 100 % de copolymère vinyl-2-pyridine-styrène (70-30).

On obtient ainsi des granulés dont les caractéristiques sont les suivantes :

| | Produit enrobé contenant de l'acétobutyrate de cellulose | Témoin |
|---|---|---|
| Titre en méthionine | 66,0 % | 62,5 % |
| Extractible dans l'eau | | |
|   - après 16 heures | 5,8 % | 7,1 % |
|   - après 48 heures | 9,1 % | 19,1 % |
| Résistance au rumen | | |
|   - après 48 heures | 90 % | 86 % |
| Extractible en milieu acide pH 1,5 | | |
|   - après 15 minutes | 58 % | 24 % |
|   - après 30 minutes | 85 % | 45 % |
|   - après 1 heure | 100 % | 47 % |
| Méthioninémie (12 g/jour x 7 jours) en mg de méthionine/100 g de sang | | |
|     Brebis  A | 12,0 | 7,1 |
|           B | 16,0 | 6,4 |
|           C | 13,3 | 3,1 |
|           D | 12,4 | 6,0 |
|           E | 10,2 | |
|           F | | 1,8 |
|           G | | 5,4 |

## Exemple 4

On enrobe 400 g de méthionine granulée sous forme de particules sphériques titrant 98 % dont le diamètre est compris entre 0,5 et 0,63 mm, selon la technique du lit fluidisé, avec 87,1 g d'un enrobage constitué de 57,9 parties de copolymère vinyl-2 pyridine-styrène (70-30), 24,8 parties d'acétobutyrate de cellulose et 17,3 parties d'acide phtalique.

On obtient des granulés dont les caractéristiques sont les suivantes :

| | |
|---|---|
| — titre en méthionine | 75 % |
| — extractible dans l'eau à 20 °C | 11,3 % après 16 heures |
| | 17,8 % après 48 heures |
| — résistance dans le rumen | 72,3 % après 48 heures |
| — extractible en milieu acide | à pH 1,5 |

42 % après 15 minutes ; 70 % après 30 minutes ; 90 % après 1 heure à pH 3
 9 % après 15 minutes ; 16 % après 30 minutes ; 20 % après 1 heure

## Exemple 5

On enrobe 400 g de méthionine granulée sous forme de particules sphériques titrant 98 % dont le diamètre est compris entre 0,5 et 0,63 mm, selon la technique du lit fluidisé, avec 80 g d'un enrobage constitué de 70 parties de copolymère vinyl-2 pyridine-styrène (70-30), 20 parties d'acétobutyrate de cellulose et 10 parties d'acide phtalique.

On obtient des granulés dont les caractéristiques sont les suivantes :

| | |
|---|---|
| — titre en méthionine | 66,3 % |
| — extractible dans l'eau | 5,2 % en 16 heures |
| | 8,3 % en 48 heures |
| — résistance au rumen | 64,0 % après 48 heures |
| — extractible en milieu acide | à pH 1,5 |

77,0 % après 15 minutes ; 95,0 % après 30 minutes ; 100 % après 1 heure à pH 3
10,5 % après 15 minutes ; 16 % après 30 minutes ; 28 % après 1 heure.

6

A titre de comparaison, un produit enrobé par le copolymère vinyl-2 pyridine-styrène (70-30) seul, dans les conditions décrites à l'exemple 1 présente un taux d'extractible à pH 3 de :
6 % après 15 minutes ; 8 % après 30 minutes ; 15 % après 1 heure.

Exemple 6

On enrobe 400 g de méthionine granulée sous forme de particules sphériques dont le diamètre est compris entre 0,5 et 0,63 mm titrant 98 % avec 72 g d'un enrobage constitué de 70 parties de copolymère vinyl-2 pyridine-styrène (90-10) et 30 parties d'acétobutyrate de cellulose, en opérant en lit fluidisé.

On obtient ainsi des granulés dont les caractéristiques sont les suivantes :

| | |
|---|---|
| — titre en méthionine | 72,2 % |
| — extractible dans l'eau | 11,8 % en 16 heures |
| | 24,5 % en 48 heures |
| — résistance dans le rumen | 85,5 % après 48 heures |
| — extractible en milieu acide | à pH 1,5 |

85,6 % après 15 minutes ; 100 % après 30 minutes ; 100 % après 1 heure à pH 3
5 % après 15 minutes ; 15 % après 30 minutes ; 26 % après 1 heure

Exemple 7

On enrobe 400 g de méthionine granulée sous forme de particules sphériques dont le diamètre est compris entre 0,5 et 0,63 mm, titrant 98 % environ avec 120 g d'un enrobage constitué d'un mélange de 67 parties d'un copolymère méthyl-2 vinyl-5 pyridine-styrène (77,4-22,6) et de 33 parties d'éthylcellulose, en opérant en lit fluidisé.

On obtient ainsi des granulés dont les caractéristiques sont les suivantes :

| | |
|---|---|
| — titre en méthionine | 66,2 % |
| — extractible dans l'eau | 11,3 % en 16 heures |
| | 20,1 % en 48 heures |
| — résistance au rumen | 84 % après 48 heures |
| — extractible en milieu acide | à pH 1,5 |

27 % après 15 minutes ; 50 % après 30 minutes ; 66 % après 1 heure

A titre de comparaison, des granulés préparés à partir de 400 g de méthionine et de 120 g de copolymère méthyl-2 vinyl-5 pyridine-styrène présentent les caractéristiques suivantes :

| | |
|---|---|
| — titre en méthionine | 62,9 % |
| — extractible dans l'eau | 14,5 % après 16 heures |
| | 20,1 % après 48 heures |
| — extractible en milieu acide | à pH 1,5 |

18 % après 15 minutes ; 35 % après 30 minutes ; 48 % après 1 heure

Le copolymère méthyl-2 vinyl-5 pyridine-styrène (77-23) peut être préparé de la manière suivante :
Dans un réacteur de 25 litres, on dissout 17,5 g de méthyl-cellulose dans 11 667 g d'eau distillée. La solution est chauffée à 60 °C puis on ajoute un mélange de 1050 g de styrène, de 3950 g de méthyl-2 vinyl-5 pyridine et de 50 g d'azobisisobutyronitrile. Après 4 heures 30 de chauffage à 63 °C tout en agitant à la vitesse de 150 tours/minute, le polymère est pris en masse. On ajoute 7 litres de diméthylformamide après avoir soutiré le liquide surnageant.

La solution obtenue est versée dans l'eau à raison de 1 litre de solution pour 10 litres d'eau. Le polymère précipité est essoré, lavé à l'eau distillée et séché à 60 °C sous pression réduite (100 mm de mercure ; 13,3 kPa) jusqu'à poids constant.

On obtient ainsi 4200 g de copolymère méthyl-5 vinyl-2 pyridine-styrène (77,3-22,6) dont la viscosité spécifique est 0,486 (détermination en solution à 5 g/litre dans le diméthylformamide à 20 °C).

Le taux de polymérisation est de 84 %.

**Revendications**

1. Composition homogène pour l'enrobage d'une substance biologiquement active qui est stable dans un milieu dont le pH est supérieur ou égal à 5 et qui permet la libération de la substance biologiquement active dans un milieu dont le pH est inférieur ou égal à 3,5 caractérisée en ce qu'elle est constituée d'un mélange homogène d'un copolymère non réticulé sensible aux variations du pH choisi parmi les copolymères du styrène avec les vinylpyridines dans lesquels la teneur en styrène est comprise entre 5 et 70 % et d'un polymère hydrophobe non hydrosoluble, insensible aux variations du pH dans lequel le copolymère non réticulé est soluble choisi parmi l'acétobutyrate de cellulose, l'éthylcellulose et le propionate de cellulose, et éventuellement d'un acide organique choisi parmi les acides phtalique, oxalique, malonique, succinique et benzoïque et d'adjuvants choisis parmi les plastifiants, les agents antistatiques et les colorants.

2. Composition selon la revendication 1 caractérisée en ce que le copolymère non réticulé est choisi parmi les copolymères styrène-vinyl-2 pyridine, styrène-vinyl-4 pyridine et styrène-méthyl-2 vinyl-5 pyridine.

3. Composition selon la revendication 1 caractérisée en ce que le polymère hydrophobe non hydrosoluble insensible aux variations du pH représente 20 à 40 % en poids du mélange des polymères.

4. Granulés destinés à être administrés par voie orale à des animaux caractérisés en ce qu'ils sont constitués d'un noyau contenant une substance biologiquement active entouré d'une pellicule d'enrobage selon la revendication 1.

5. Granulés selon la revendication 4 caractérisés en ce que la substance biologiquement active est la méthionine.

## Claims

1. Homogeneous composition for coating a biologically active substance, which is stable in a medium with a pH greater than or equal to 5 and which enables the biologically active substance to be released in a medium with a pH less than or equal to 3.5, characterized in that it consists of a homogeneous mixture of an uncrosslinked copolymer which is sensitive to pH variations, chosen from amongst copolymers of styrene with vinyl-pyridines, in which the styrene content is between 5 and 70 %, and a water-insoluble hydrophobic polymer which is insensitive to pH variations, in which the uncrosslinked copolymer is soluble, chosen from amongst cellulose acetobutyrate, ethylcellulose and cellulose propionate, and optionally an organic acid chosen from amongst phthalic, oxalic, malonic, succinic and benzoic acids and adjuvants chosen from amongst plasticizers, antistatic agents and colouring agents.

2. Composition according to Claim 1, characterized in that the uncrosslinked copolymer is chosen from amongst styrene/2-vinylpyridine, styrene/4-vinylpyridine and styrene/2-methyl-5-vinylpyridine copolymers.

3. Composition according to Claim 1, characterized in that the water-insoluble hydrophobic polymer which is insensitive to pH variations represents 20 to 40 % by weight of the polymer mixture.

4. Granules intended for oral administration to animals, characterized in that they consist of a nucleus containing a biologically active substance surrounded by a coating film according to Claim 1.

5. Granules according to Claim 4, characterized in that the biologically active substance is methionine.

## Patentansprüche

1. Homogene Masse zur Umhüllung einer biologisch aktiven Substanz, welche Masse in einem Milieu stabil ist, dessen, pH-Wert höher oder gleich 5 ist, und welche die Freisetzung der biologisch aktiven Substanz in einem Milieu, dessen pH-Wert kleiner oder gleich 3,5 ist, gewährleistet, dadurch gekennzeichnet, daß die Masse aus einer homogenen Mischung eines unvernetzten, gegenüber Änderungen des pH-Wertes empfindlichen Copolymers ausgewählt aus den Copolymeren von Styrol mit den Vinylpyridinen, wobei der Styrolgehalt zwischen 5 und 70 % ist, und eines hydrophoben, wasserunlöslichen, gegenüber Änderungen des pH-Wertes unempfindlichen Polymers, in welchem das unvernetzte Copolymer löslich ist, ausgewählt aus Celluloseacetobutyrat, Äthylcellulose und Cellulosepropionat, und gegebenenfalls einer organischen Säure, ausgewählt aus Phthal-, Oxal-, Malon-, Bernstein- und Benzoesäure, sowie Zusatzstoffen, ausgewählt aus Plastifizierungsmitteln, antistatischen Mitteln und Farbstoffen, besteht.

2. Masse nach Anspruch 1, dadurch gekennzeichnet, daß das unvernetzte Copolymere ausgewählt ist aus den Copolymeren Styrol-2-vinylpyridin, Styrol-4-vinylpyridin und Styrol-2-methyl-5-vinylpyridin.

3. Masse nach Anspruch 1, dadurch gekennzeichnet, daß das hydrophobe, wasserunlösliche, gegenüber Änderungen des pH-Wertes unempfindliche Polymer 20 bis 40 Gew.-% der Polymermischung ist.

4. Zur oralen Verabreichung an Tiere bestimmte Granalien, dadurch gekennzeichnet, daß sie aus einem, eine biologisch aktive Substanz enthaltenden Kern, der mit einer Umhüllungsschicht nach Anspruch 1 umgeben ist, besteht.

5. Granalien nach Anspruch 4, dadurch gekennzeichnet, daß die biologisch aktive Substanz Methionin ist.